# EUROPÄISCHE PATENTSCHRIFT

(11) **EP 4 190 308 B1**
(45) Veröffentlichungstag und Bekanntmachung des Hinweises auf die Patenterteilung: **23.07.2025**
(21) Anmeldenummer: 22157896.6
(22) Anmeldetag: 22.02.2022
(51) Int. Cl.: A61K 9/00, A61K 9/08, A61K 9/12, A61K 9/70, A61K 31/357, A61P 25/00, A61P 25/22, A61P 25/24

(54) **SCHLAFDUFT SOWIE HERSTELLUNG UND VERWENDUNG VON DIESEM**
SLEEPING FRAGRANCE AND ITS MANUFACTURE AND USE
PARFUM DE SOMMEIL, AINSI QUE SA PRODUCTION ET SON UTILISATION

(30) Priorität: 06.12.2021 DE 102021132026
(43) Veröffentlichungstag der Anmeldung: 07.06.2023
(73) Patentinhaber: Marome GmbH, 40212 Düsseldorf (DE)
(72) Erfinder: FELDMANN, Martin, 40212 Düsseldorf (DE); FELDMANN, Arne, 8712 Stäfa (CH)
(74) Vertreter: Gille Hrabal Partnerschaftsgesellschaft mbB Patentanwälte

(56) Entgegenhaltungen:
- EP-A1- 0 982 303
- DE-A1- 102007 010 077
- DE-A1- 3 429 227
- JP-A- 2005 290 379
- KR-A- 20200 102 038
- US-A1- 2021 008 082
- SERGEEVA OLGA A ET AL: "Fragrant Dioxane Derivatives Identify beta1-Subunit-containing GABA A Receptors", vol. 285, no. 31, 28 May 2010 (2010-05-28), pages 23985 - 23993, XP055967950, Retrieved from the Internet <URL:https://www.jbc.org/article/S0021-9258(20)61865-8/pdf> [retrieved on 20221003], DOI: 10.1074/jbc.M110.103309

## Beschreibung

Die vorliegende Anmeldung betrifft eine Zusammensetzung, umfassend eine Verbindung gemäß Formel (I): 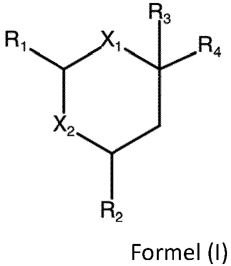 und ein ätherisches Lösungsmittel, wobei die erfindungsgemäße Zusammensetzung 0,1 bis 5 Gew.-% 2,4,6-Trimethyl-4-phenyl-1,3-dioxan und 1 bis 50 Gew.-% Lavendelöl als ätherisches Lösungsmittel umfasst. Gegenstand der vorliegenden Erfindung ist die Herstellung einer Zusammensetzung und deren Verwendung bevorzugt zu Unterstützung des Schlafprozesses.

### STAND DER TECHNIK

Beruhigungsmittel (Sedativa), Schlafmittel (Hypnotika) und Entspannungsmittel (Tranquilizer) sind nach wie vor die häufigsten verordneten Psychopharmaka. Fast ein Drittel der deutschen Bevölkerung hat Probleme bezüglich des Einschlafens und/oder Durchschlafens. Es gibt eine Reihe von Medikamenten und frei verkäuflichen Mitteln zum Einnehmen als Unterstützung des Schlafprozesses. Die bisher bekannten und/oder verschriebenen Mittel/Medikamente können jedoch häufig zu Abhängigkeiten führen. Besonders häufig führen Mittel, die eingeatmet werden müssen, zu unerwünschten Abhängigkeiten. Andere Mittel sind nicht wirksam oder müssen intravenös, parental oder oral verabreicht werden.

Inzwischen sind verschiedene Gruppen von Pharmaka bekannt, die zentral dämpfend, d. h. auf die Aktivität des Aktivierungszentrums in der *Formatio reticularis* vermindernd wirken. Die klassischen Stoffklassen, von denen sedative/hypnotische Wirksamkeit bekannt ist, sind Alkohole, Barbiturate, halogenierte Kohlenstoffverbindungen, Opiate (Morphinderivate), Promide und Promoreide. Mitte der fünfziger Jahre wurde zusätzlich eine Gruppe von Arzneimittelwirkstoffen entdeckt, die unter dem Oberbegriff Benzodiazepine heute zu den weltweit am häufigsten verordneten Arzneimitteln gehören. Sie zeichnen sich durch eine angstlösende, beruhigende, schlaffördernde und erregungsdämpfende Wirkung aus. Neben der starken Suchtgefahr wurden allerdings im Laufe der Jahrzehnte eine Reihe von ernstzunehmenden Nebenwirkungen registriert, die sich vor allem in Benommenheit, depressiver Verstimmung und zum Teil paradoxen Reaktionen (akute Erregungszustände), aber auch körperlichen Symptomen, wie Blutdruckabfall, Atembeschwerden, Mundtrockenheit, Muskelschwäche und Koordinationsstörungen mit verlängerter Reaktionszeit ausdrücken.

Benzodiazepine wirken über spezifische Haftstellen an Rezeptoren, die an Kontaktstellen von Nervenzellen (Synapsen) liegen und die Aktivität des hemmenden Neurotransmitters GABA verstärken. Es existiert entsprechend ein enger Zusammenhang zwischen Benzodiazepinen und GABA-Rezeptoren. Eine agonistische Besetzung der Bindestelle für Benzodiazepine an GABA-Kanalproteinen führt zur Erhöhung der Affinität des GABA-Rezeptors, dadurch wird die für das GABA-Kanalprotein typische Wirkung gesteigert. Ähnliche Wirkprinzipien am GABA-Rezeptorkanalkomplex haben auch Barbiturate oder das kurz wirkende Hypnotikum (Anästhetikum) Propofol. Beide besitzen ebenfalls Bindestellen am GABA-Kanalprotein und verstärken die Wirkung des hemmenden Neurotransmitters GABA. Diese "allosterischen" Effektoren der GABA-Rezeptoren sind selbst nur in extrem hohen Konzentrationen in der Lage, den GABA-Kanal zu aktivieren, allerdings steigern sie die Wirkung des hemmenden Neurotransmitters um das 2-3fache. In Deutschland nehmen bis zu 20% der Bevölkerung im Verlauf eines Jahres GABAerge Modulatoren zur Behandlung von Spannungs-, Erregungs- und Angstzuständen, Schlafstörungen, als sog. Tranquilizer oder Psychopharmaka ein oder werden im Rahmen von Kurzzeitnarkosen klinisch damit behandelt.

EP 0 288 279 A und DE 34 29 227 A beschreiben 1,3-Dioxanderivate, Verfahren zur Herstellung dieser 1,3-Dioxanderivate.

EP 0 288 279 A offenbart insbesondere die Verwendung von Säurederivaten von 1,3-Dioxolanen sowie pharmazeutische Zusammensetzungen zur therapeutischen Verwendung bei Krankheiten, wie ischämischen Herzerkrankungen, zerebrovaskulären Erkrankungen, asthmatischen und/oder Entzündungskrankheiten.

DE 34 29 227 A offenbart die Verwendung der 1,3-Dioxanderivate als Arzneimittel gegen *Ulcus pepticum.*

DE 10 2007 010 077 A offenbart zusätzlich die Modulationsmöglichkeiten des GABA-Rezeptors mittels 1,3-Dioxanderivate insbesondere zur Therapie von Depressionen, Unruhe- und Angstzuständen, Schlafstörungen, Epilepsie, Demenzerkrankungen, Alkoholismus und/oder Migräne. Speziell werden in diesem Zusammenhang verschiedene Applikationsmöglichkeiten wie Inhalation offenbart.

EP 0 982 303 A1 beschreibt Verbindungen der allgemeinen Formel (2RS,4SR,6X)-2,4,6-Trimethyl-4-phenyl-1,3-dioxan, die einen Geruch besitzen, der sich als stark, krautig-frisch, grün, typisch Grapefruit beschreiben lässt, wobei auch Zusammensetzungen mit einer Vielzahl von unterschiedlichen Bestandteilen, darunter auch manche ätherische Öle, offenbart werden.

US 2021/008082 A1 offenbart Duftstoffzusammensetzungen, die eine oder mehrere Duftstoffverbindungen zur Verwendung bei der Verlängerung der Schlafdauer und/oder der Verbesserung der Schlafqualität bei einem Subjekt umfassen, wobei KR 2020 0102038 A Duftkompositionen zur Spannungslinderung und Konzentrationssteigerung beschreibt.

JP 2005 290379 A betrifft Parfümzusammensetzungen, die einem Benutzer körperliches und geistiges Wohlbefinden bieten sollen, indem die therapeutische Kraft des Aromas gegen physiologische oder psychologische Symptome genutzt wird.

Der wissenschaftliche Artikel SEGREEVA OLGA A ET AL: "Fragrant Dioxane Derivates Identify β1-Subunit-containing GABAA Receptors", THE JOURNAL OF BIOLOGICAL CHEMISTRY (Bd. 285, Nr.31, 28. Mai 2010) lehrt, dass der Hypothalamus ein Zentrum für den Schlafen-Aufwach-Rhythmus ist, und dass die 2,4,6-Trimethyl-4-phenyl-1,3-dioxan Verbindung das Schlafverhalten günstig beeinflusst. Eine Kombination mit ätherischen Ölen ist jedoch nicht beschrieben. KR 2020 0102038 A offenbart Duftkompositionen, die in Lebensmitteln und Kosmetika zur Linderung von Verspannungen und zur Steigerung der Konzentration nützlich sind und Floropal^{®} (2,4,6-Trimethyl-4-phenyl-1,3-dioxan), Linalool, Ethyllinalool, Linalylacetat, Orangenöl und andere Komponenten enthalten.

Die vorstehenden Offenbarungen beschreiben jedoch keine angepassten Wirkungsbereiche der Benzodiazepine, speziell der 1,3-Dioxanderivate, die einen möglichst hohen Effekt bei möglichst niedriger Dosierung ermöglichen. Speziell werden keine unterstützenden oder synergistischen Effekte durch andere Komponenten vorgeschlagen, die zu einer verbesserten Wirksamkeit speziell bei Schlafstörungen führen und im Einzelnen eine Wirkung nicht nur für den Einschlafprozess, sondern auch den Durchschlafprozess aufweisen.

Die vorliegende Erfindung stellt sich ausgehend von diesem Stand der Technik zunächst einmal eine erste Aufgabe, eine Zusammensetzung bereitzustellen, die einen angenehmen Duft aufweist und für einen angenehmen Einschlafprozess dienen kann. Dabei sollte der Duft insgesamt nicht zu aufdrängend ausgebildet sein und ein angenehmes Riechgefühl bei den Probanden erzeugen.

Diese Aufgabe stellt sich die vorliegende Erfindung insbesondere außerhalb jedweder arzneirechtlichen Überlegung; der erfindungsgemäß bereitzustellende Duft bedarf keiner arzneimittelrechtlichen Wirkung, sondern soll insgesamt nur ein angenehmes Geruchsprofil gegenüber Probanden aufweisen, so dass diese mit einem angenehmen Gefühl einschlafen können.

Zusätzlich stellt sich die vorliegende Erfindung auch die Aufgabe, eine Zusammensetzung bereitzustellen zur Erreichung eines erhöhten Wirkungsgrades im Verhältnis zur notwendigen Menge an der erfindungsgemäßen Verbindung bei der Behandlung von verschiedenen körperlichen und/oder psychischen Einschränkungen, wie beispielsweise Unruhe- und Angstzustände sowie Schlafstörungen, zu ermöglichen.

Der Erfindung liegt weiterhin die Aufgabe zugrunde, mögliche Abhängigkeiten durch die Einnahme von Verbindungen der Klasse der Benzodiazepine zu reduzieren.

Der Erfindung liegt weiterhin die Aufgabe zugrunde, eine möglichst vorteilhafte Applikationsmöglichkeit anzubieten, die auf breite Akzeptanz unter den Anwendern stößt und/oder möglichst nicht invasiv ist. Insbesondere liegt der Erfindung die Aufgabe zugrunde, dem Anwender ein angenehmes und positives Gefühl bei der Verwendung der Zusammensetzung zu ermöglichen.

Der Erfindung liegt weiterhin die Aufgabe zugrunde, die Kosten bei der Herstellung der Zusammensetzung zu verringern.

Zur Lösung einer oder mehrerer der genannten Aufgaben dient eine Zusammensetzung, umfassend 0,1 bis 5 Gew.-% 2,4,6-Trimethyl-4-phenyl-1,3-dioxan und 1 bis 50 Gew.-% Lavendelöl als ätherisches Lösungsmittel.

Die beanspruchte Mischung der beiden erfindungsgemäß vorgesehenen Bestandteile weist vorzugsweise ein spezifisches Verhältnis auf und/oder ermöglicht eine bevorzugte Applikation.

Entsprechende Ausführungsformen sind in den unabhängigen Patentansprüchen sowie den abhängigen Patentansprüchen definiert.

Zur Lösung einer oder mehrerer der genannten Aufgaben dient auch die Herstellung der spezifischen Wirkstoffkombination durch Mischen der Bestandteile der erfindungsgemäßen Zusammensetzung.

Zur Lösung einer oder mehrerer der genannten Aufgaben dient zusätzlich die Verwendung der spezifischen erfindungsgemäßen Wirkstoffkombination.

### KURZBESCHREIBUNG DER ERFINDUNG

[1,3]-Dioxane als positive allosterische Modulatoren besitzen eine Spezifität für GABA_{A}-Rezeptoren, die β1-Untereinheiten enthalten und sind daher für alle Anwendungen interessant, die auf der Beeinflussung von GABA_{A}-Rezeptoren beruhen. Speziell können die Substanzen eingesetzt werden, um sedierende, angstlösende oder betäubende Wirkungen auszulösen, welche auf die Potenzierung von GABA_{A}-Rezeptoren ansprechen.

Es wurde nunmehr überraschend gefunden, dass bestimmte [1,3]-Dioxan-Derivate und akzeptable Salze als allosterischen Modulatoren der GABA-Wirkung ab einer bestimmten Menge zu keinem höheren erwünschten Effekt führen, sondern viel mehr ein Effektplateau erreicht wird. Es wurde darüber hinaus überraschend gefunden, dass eine Zusammensetzung mit zusätzlichen ätherischen Lösungsmitteln einen synergistischen Effekt auslösen. Speziell wurde ein sehr viel höherer Wirkungsgrad erzielt, als der Fachmann durch die Menge an [1,3]-Dioxan-Derivate erwartet hätte.

Als Konsequenz der verminderten Menge an Verbindung gemäß der Formel (I) können hierdurch andere Stoffe in die erfindungsgemäße Zusammensetzung eingeführt werden, die einen zusätzlichen Zweck erfüllen, oder den gewünschten Effekt verstärken, beispielsweise über einen anderen Wirkmechanismus.

Speziell können hier beispielsweise ätherische Lösungsmittel verwendet werden, die einerseits der Zusammensetzung einen verbesserten Duft geben, sodass die erfindungsgemäße Zusammensetzung angenehm zum Einatmen auch über einen längeren Zeitraum ist. Darüber hinaus können diese ätherischen Lösungsmittel auch schlaffördernde und/oder sedierende und/oder betäubende und/oder angstlösende Effekte als Unterstützung der erfindungsgemäßen Verbindung aufweisen. Weiterhin können die zusätzlichen Komponenten, wie die ätherische Lösungsmittel, einen oder mehrere weitere Effekte unabhängig von der erfindungsgemäßen Verbindung erfüllen, wie beispielsweise eine verlängerte Konservierungsmöglichkeit.

Weiterhin reduziert die erfindungsgemäße Zusammensetzung das Risiko unerwünschter Abhängigkeiten. Speziell werden vor allem körperliche Abhängigkeiten durch eine verminderte allosterische Stimulation der GABA_{A}-Rezeptors durch die Reduzierung beispielsweise der Menge der erfindungsgemäßen Verbindung vermindert.

Darüber hinaus können anhand der reduzierten notwendigen Menge an der erfindungsgemäßen Verbindung auch Herstellungskosen reduziert werden.

### DETAILLIERTE BESCHREIBUNG DER ERFINDUNG

Zur Lösung der Aufgabe dient eine Zusammensetzung, umfassend eine Verbindung gemäß folgender Formel (I): 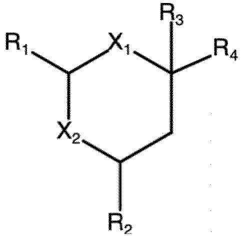 und Lavendelöl als ätherisches Lösungsmittel, wobei
R₁ ein Methyl-Rest ist,
R₂ ein Methyl-Rest ist,
R₃ ein Methyl-Rest ist,
R₄ einem Phenyl entspricht,
X₁ O entspricht,
X₂ O entspricht.
Somit dient zur Lösung der Aufgabe eine Zusammensetzung, umfassend konkret 0,1 bis 5 Gew.-% 2,4,6-
Trimethyl-4-phenyl-1,3-dioxan und 1 bis 50 Gew.-% Lavendelöl als ätherisches Lösungsmittel.

Die erfindungsgemäße Zusammensetzung definiert in einer Ausführungsform die Gesamtheit der Zusammensetzung, umfassend alle Komponenten, Inhaltsstoffe, Träger, Lösungsmittel und ähnliches die in der Zusammensetzung vorhanden sind.

Die folgende Beschreibung der bevorzugten Ausführungsform(en) ist lediglich beispielhaft und soll in keiner Weise die Erfindung, ihre Anwendung oder Verwendung einschränken. Weitere vorteilhafte Ausführungsformen ergeben sich auch aus den Unteransprüchen. Es ist dem Fachmann bewusst, dass die verschiedenen Ausführungsformen und/oder bevorzugten Beschreibungen und/oder Aspekte der Beschreibung miteinander kombiniert werden können.

In der gesamten Beschreibung werden Bereiche als Abkürzung für die Beschreibung jedes einzelnen Wertes verwendet, der innerhalb des Bereichs liegt. Jeder beliebige Wert innerhalb des Bereichs kann als Endpunkt des Bereichs gewählt werden. Darüber hinaus werden alle hierin zitierten Verweise hiermit in ihrer Gesamtheit einbezogen. Im Falle eines Widerspruchs zwischen einer Definition in der vorliegenden Offenbarung und einer Definition in einer zitierten Referenz ist die vorliegende Offenbarung maßgeblich. Hierbei sind insbesondere die Werte umfasst, die den Verhältnissen der erfindungsgemäßen Zusammensetzungen gemäß den getesteten Bedingungen 2 und 3 aus den Beispielen entsprechen, da besonders für diese Verhältnisse der erfindungsgemäße Effekt über die gesamte Breite gezeigt wurde.

Sofern nicht anders angegeben, beziehen sich alle hier und an anderer Stelle in der Beschreibung genannten Prozentsätze und Mengen auf Gewichtsprozente. Die angegebenen Mengen beziehen sich auf das aktive Gewicht des Materials.

Wie sie in dieser Beschreibung verwendet werden, umfassen die Singularformen "ein(e)", "ein(er)" und "der", "die", "das" die jeweilige Mehrzahl, falls sich nicht aus dem Zusammenhang eindeutig etwas anderes ergibt. Folglich umfasst z.B. eine Bezugnahme auf bspw. ein "ätherisches Lösungsmittel" eine einzelne Art eines ätherischen Lösungsmittels oder zwei oder mehr verschiedene Arten von ätherischen Lösungsmitteln.

Die erfindungsgemäße Zusammensetzung unterstützt die GABA induzierte hemmende Kanalströme stark zu potenzieren. Diese Verbindungen wirken vorzugsweise nicht nur bei systemischer intravenöser oder parenteraler Applikation, sondern auch bei oraler Aufnahme und besonders bevorzugt bei Inhalation oder Einatmen.

In einer Ausführungsform unterstützt die erfindungsgemäße Zusammensetzung angstlösende, beruhigende, erregungs- und aggressionsdämpfende, schlafanstoßende und/oder schlaferhaltende Anwendungen. Bevorzugt unterstützt die erfindungsgemäße Zusammensetzung schlafanstoßende und/oder schlaferhaltende Anwendungen.

Durch zusätzliche Komponenten können noch besser wirksame erfindungsgemäßen Zusammensetzungen und/oder Verwendungen der erfindungsgemäßen Zusammensetzung mit einer anwendungsfreundlicheren und/oder anwendungsübergreifenden Breite entwickelt werden.

Die erfindungsgemäße Verwendung nutzt unter anderem die Wirkung von bestimmten 1,3-Dioxanderivaten auf GABA-Kanalproteine. Diese Verbindungen induzieren eine dosisabhängige Potenzierung der GABAergen Wirkung. Die Effekte sind zum Teil stärker als die der seit langem bekannten Benzodiazepine. Es ist bekannt, dass die Wirkung von GABA in Gegenwart von 1,3-Dioxanderivaten bis zu 15-fach erhöht sein kann. Diese Potenzierung der GABA-Antwort kann z. B. eine Sedierung, Anxiolyse oder Anästhesie bewirken oder zu anderen, für GABA-potentzierter typischer Wirkung führen. Diese Substanzen haben eine Spezifität für GABA-Rezeptoren, welche eine β1-Untereinheit enthalten, wirken aber im verminderten Maße auch auf Rezeptoren mit β2- oder β3-Untereinheiten. Sie sind also im besonderen Masse geeignet, Gehirnbereiche zu beeinflussen, die eine hohe Dichte an GABA-Rezeptoren im Allgemeinen und an β1-enthaltenden GABA-Rezeptoren im speziellen aufweisen.

Erfindungsgemäß wird die Verbindung 2,4,6-Trimethyl-4-phenyl-1,3-dioxan verwendet, die eine hohe Spezifität für GABA_{A}-Rezeptoren haben, die eine β1-Untereinheit enthalten, sodass eine Modulation mit anderen Wirkstoffen, bevorzugt über andere Mechanismen, noch immer entsprechend gut möglich ist.

Erfindungsgemäß kann das ätherische Lösungsmittel eine oder mehreren Komponenten umfassen und ist nicht ausschließlich auf ätherische Lösungsmittel beschränkt, kann aber bevorzugt nur aus ätherischen Lösungsmitteln und Mischungen aus diesen bestehen.

Erfindungsgemäß handelt es sich bei "Ölen" um organische Flüssigkeiten. Bei fetten Ölen handelt es sich um Fette, also Gemische von Fettsäuretriglyceriden, die bei Raumtemperatur flüssig sind. Typische fette Öle im Sinne der Erfindung sind entweder pflanzliche Öle, wie etwa Leinöl, Sonnenblumenöl, Olivenöl, Distelöl, Hanföl, Rapsöl oder Sojaöl, oder tierische Öle, wie etwa Lebertran oder Fischöl.

Ätherische Lösungsmittel im Sinne der Erfindung umfassen verschiedene, ineinander lösliche, organische Stoffe wie beispielsweise Alkohole, Ester, Ketone, Terpene und/oder Mischungen aus diesen, besonders bevorzugt ätherische Öle und Mischungen aus ätherischen Ölen. Ätherische Öle können synthetisch oder aus natürlichen Quellen durch Wasserdampfdestillation, Extraktion oder Auspressen der Pflanzen oder der Pflanzenteile gewonnen werden. Natürliche oder naturbelassene ätherische Öle werden größtenteils, bevorzugt komplett aus sekundären Pflanzeninhaltsstoffen gewonnen und bestehen größtenteils, bevorzugt komplett aus Gemischen verschiedener Terpene, Sesquiterpene oder aromatischer Verbindungen (z. B. Phenylpropan-Derivate). Terpene leiten sich formal aus Isopren Einheiten ab. Monoterpene bestehen aus zwei, Sesquiterpene aus drei Isopren Einheiten. Ätherische Öle können fettlöslich sein, aber keine Fette enthalten. Diese fettfreien ätherischen Öle sind um einiges länger haltbar, da sie weniger schnell verderben. Das Verderben pflanzlicher und tierischer Fette, das schon im Anfangsstadium durch Geruchs- und Geschmacksänderung (Ranzigkeit) wahrgenommen werden kann, ist auf eine Hydrolyse und damit einhergehende Spaltung langkettiger Fette und zum anderen auf die Einwirkung von Luftsauerstoff (Oxidation) zurückzuführen.

Bevorzugt ist eine Ausführungsform der erfindungsgemäßen Zusammensetzung, bei der das ätherische Lösungsmittel, oder eine Mischung aus ätherischen Lösungsmitteln solche umfasst, die fettlöslich sind, aber kein Fett enthalten, bevorzugter aus solchen besteht, die die fettlöslich sind, aber kein Fett enthalten, um eine längere Haltbarkeit und verminderte Geruchsbelästigung nach einer gewissen Zeit zu gewährleisten.

Bevorzugt ist eine Ausführungsform der erfindungsgemäßen Zusammensetzung, bei der das ätherische Lösungsmittel, oder eine Mischung aus ätherischen Lösungsmitteln keine umfasst, die Fett enthalten, um ebenfalls eine längere Haltbarkeit und verminderte Geruchsbelästigung nach einer gewissen Zeit zu gewährleisten.

In einer zusätzlichen Ausführungsform der erfindungsgemäßen Gesamtmischung umfasst das erfindungsgemäße ätherische Lösungsmittel naturbelassene und/oder natürliche und/oder naturidentische und/oder synthetische ätherische Öle. Naturbelassene ätherische Öle im Sinne der Erfindung werden direkt aus Pflanzen bevorzugt in Form von sekundären Pflanzenstoffen gewonnen. Natürliche ätherische Öle im Sinne der Erfindung bestehen aus naturbelassenen Ölen mit anderen Zusätzen. Naturidentische ätherische Öle im Sinne der Erfindung bestehen aus den wichtigsten, merkmalgebenden Stoffen/Komponenten eines ätherischen Öls. Synthetische ätherische Öle werden gezielt auf den Geruch hin entworfen und kommen nicht in der Natur vor. Der Vorteil bei der Verwendung von synthetischem Öl ist die kostengünstige Herstellung und sehr gute Verträglichkeit aufgrund der geringen und manipulierbaren Inhaltsstoffe. Der Vorteil bei der Verwendung von naturidentischen ätherischen Ölen ist ebenfalls eine kostengünstige Herstellung, die jedoch aufgrund des Inhalts der wichtigsten Stoffe/Komponente aus den sekundären Pflanzenstoffen hinsichtlich spezifischer Merkmale vor allem hinsichtlich des Geruchs, aber vermindert auch in anderen Wirkungen verbessert gegenüber der Verwendung von synthetischen ätherischen Ölen ist. Der Vorteil bei der Verwendung von natürlichen ätherischen Ölen ist der komplette Zugang an sekundären Pflanzenstoffen der Pflanze, die jedoch zusätzlich weitere Inhaltsstoffe wie beispielsweise Konservierungsstoffe enthalten können und so das natürliche ätherische Öl in bestimmten Merkmalen verbessern können, wie zum Beispiel bezüglich der Haltbarkeit, während die typischen Merkmale und besonders der Geruch größtenteils unverfälscht erhalten bleibt. Der Vorteil bei der Verwendung von naturbelassenem ätherischem Öl ist der komplette und am ehesten unverfälschten Erhalt der spezifischen Merkmale wie Wirkung und Geruch aus den sekundären Pflanzenstoffen.

In einer Ausführungsform umfasst die erfindungsgemäße Zusammensetzung mind. ein natürliches und/oder naturbelassenes ätherisches Öl oder Mischungen aus diesen als ätherisches Lösungsmittel neben anderen Komponenten. Bevorzugt hat das natürliche und/oder naturbelassene ätherische Öl oder Mischungen aus diesen den größten Anteil in dem ätherischen Lösungsmittel. Durch die Verwendung von natürlichem und/oder naturbelassenem ätherischem Öl oder Mischungen aus diesen kann eine verstärkte Wirkung durch das gesamte Spektrum der typischen sekundären Pflanzeninhaltsstoffe der Pflanzensorte, sowie ein unverfälschter Duft gewährleistet werden.

In einer Ausführungsform umfasst die erfindungsgemäße Zusammensetzung mind. ein synthetisches ätherisches Öl als ätherisches Lösungsmittel. Bevorzugt hat das mind. ein synthetisches ätherisches Öl den größten Anteil in dem ätherischen Lösungsmittel. Durch die Verwendung von synthetischem ätherischem Öl kann durch die reduzierte Menge an Inhaltsstoffen eine höhere Verträglichkeit gewährleistet werden.

In einer anderen Ausführungsform umfasst die erfindungsgemäße Zusammensetzung entweder ausschließlich mind. ein erfindungsgemäßes synthetisches ätherisches Öl für das erfindungsgemäße ätherische Lösungsmittel oder wird komplett ausgeschlossen.

In einer anderen Ausführungsform umfasst die erfindungsgemäße Zusammensetzung entweder ausschließlich mind. ein erfindungsgemäßes naturidentisches ätherisches Öl für das erfindungsgemäße ätherische Lösungsmittel oder wird komplett ausgeschlossen.

In einer anderen Ausführungsform umfasst die erfindungsgemäße Zusammensetzung entweder ausschließlich mind. ein erfindungsgemäßes natürliches ätherisches Öl für das erfindungsgemäße ätherische Lösungsmittel oder wird komplett ausgeschlossen.

In einer anderen Ausführungsform umfasst die erfindungsgemäße Zusammensetzung entweder ausschließlich mind. ein erfindungsgemäßes naturbelassenes ätherisches Öl für das erfindungsgemäße ätherische Lösungsmittel, oder wird komplett ausgeschlossen. Speziell ermöglicht die Verwendung einer spezifischen ätherischen Öl Art ausgewählt aus naturbelassenen, natürlichen, naturidentischen bzw. synthetischen ätherischen Ölen eine verbesserte Kontrolle der Inhaltsstoffe und Modulation der Wirkung der Zusammensetzung.

In einer anderen Ausführungsform ist mind. ein weiterer Inhaltsstoff in der erfindungsgemäßen Zusammensetzung umfasst.

In einer weiteren Ausführungsform ist mind. ein Träger in der erfindungsgemäßen Zusammensetzung umfasst. Träger sind Stoffe, welche die erfindungsgemäßen Pflanzeninhaltsstoffe, ätherischen Lösungsmittel, die Verbindung 2,4,6-Trimethyl-4-phenyl-1,3-dioxan und andere Komponenten in der erfindungsgemäßen Zusammensetzung binden, die Zusammensetzung konservieren und/oder bei Anwendung für eine gleichmäßige Verteilung Und/oder eine Wirkungsentfaltung gewährleisten. Wasser, insbesondere destilliertes Wasser, kann als akzeptabler Träger fungieren.

In jedem der vorangegangenen Aspekte der Ausführungsformen befindet sich die erfindungsgemäße Zusammensetzung aufgebracht auf einem Tuch oder einem Nasenclip oder in einer Sprühvorrichtung.

In einer bevorzugten Ausführungsform befindet sich die erfindungsgemäße Zusammensetzung bevorzugt auf einem Tuch, bevorzugter auf einem Vliestuch.

Das Tuch mit der erfindungsgemäßen Zusammensetzung ist bevorzugt aus Vliesstoff, da die erfindungsgemäße Gesamtmischung in der Vliesstoffmatrix besonders gut über langen Zeitraum bewahrt und bei Verwendung gleichmäßig abgeben werden kann. Darüber hinaus fühlen sich die Anwender mit einer Verwendung mittels Schlaftuch bzw. mit der Benetzung der erfindungsgemäßen Zusammensetzung auf Pyjama oder Kopfkissen am wohlsten, wie in den Beispielen verdeutlicht. Entsprechend wird auch eine orale Applikation bevorzugt ausgeschlossen. Zusätzlich wird auch eine intravenöse und/oder parenterale Applikation bevorzugt ausgeschlossen.

Weiterhin wird auch bevorzugt eine Inhalation der erfindungsgemäßen Zusammensetzung ausgeschlossen. Inhalation im Sinne der Erfindung umfasst eine einmalige oder mehrmalige Aufnahme über die Lunge innerhalb eines kurzen Zeitraums. Bevorzugt wird die erfindungsgemäße Zusammensetzung über Einatmen aufgenommen. Einatmen im Sinne der Erfindung ist eine kontinuierliche, bevorzugt in Konzentration gleichbleibende Aufnahme über einen längeren Zeitraum. Ein längerer Zeitraum im Sinne der Erfindung umfasst mindestens 20 min, bevorzugt mindestens 1 h, bevorzugter mindestens 2 h, bevorzugter mindestens 3 h, bevorzugter mindestens 4 h, bevorzugter mindestens 5h, am bevorzugtesten über die gesamte Schlafdauer. Durch die bevorzugt kontinuierliche Applikation über einen langen Zeitraum tritt der erfindungsgemäße Effekt bzw. die erfindungsgemäßen Effekte auch bei geringeren Konzentrationen ein. Bei einer Inhalation wie oben beschrieben muss die Konzentration aufgrund der einmaligen Applikation um einiges höher sein. Speziell fördert die einmalige Applikation körperliche sowie psychische Abhängigkeiten von Medikamenten. Weiterhin fördern einmalige Applikation nicht den Erhalt des Schlafes.

In einer anderen Ausführungsform befindet sich die erfindungsgemäße Zusammensetzung auf einem Nasenclip oder einem Nasentape, da die erfindungsgemäße Gesamtmischung über den Nasenclip durch die nahe Lokation zur Nase besonders gut aufgenommen werden kann und ein Verrutschen des Nasenclips oder Nasentapes auch bei Bewegungen im Schlaf minimiert ist. Auf diese Weise ist eine dauerhafte, gleichbleibende Aufnahme der erfindungsgemäßen Zusammensetzung gewährleistet.

In einer weiteren Ausführungsform ist die erfindungsgemäße Zusammensetzung in einer Sprühflasche vorgesehen und wird von dem Anwender auf den Pyjama, Kissen, Tuch etc. aufgetragen. Hierdurch kann der Anwender die gewünschte Menge selbstständig dosieren.

In einer weiteren Ausführungsform soll die erfindungsgemäße Zusammensetzung als Crème oder Lotion auf die Haut aufgetragen werden für Personen, die eine solche Applikation bevorzugen.

In jedem der vorangegangenen Aspekte der Ausführungsformen ist die Verbindung 2,4,6-Trimethyl-4-phenyl-1,3-dioxan in einem Bereich zwischen 0,1 und 5 Gew.-% vorhanden, bezogen auf das Gesamtgewicht der erfindungsgemäßen Zusammensetzung.

Ein Aspekt der Offenbarung ist das ätherische Öl, oder die Mischung aus ätherischen Ölen für das ätherische Lösungsmittel ausgewählt aus einer Liste bestehend aus ätherischen Lösungsmitteln auf Basis von Lavendel, Thymian, Zimt, Schokolade, Vanille, Jasmin, Baldrian, Melisse, Hafer, Hopfen, Gardenia Acetal, Passionsfrucht, Rose, Zitrone, Grapefruit, Apfel, Orange, Mandarine, Birne, Sonnenblume, Kirsche, Melone, Mango, Maracuja, Kiwi, Banane, Drachenfrucht, Erdbeere, Mandel, Walnuss, Macadamianuss, Cashew und/oder Haselnuss. Erfindungsgemäß umfasst die Zusammensetzung 1 bis 50 Gew.-% Lavendelöl als ätherisches Lösungsmittel.

In einer bevorzugten Ausführungsform umfasst das ätherische Lösungsmittel Lavendelöl und Passionsblumenöl (Passiflora incarnata).

In einer Ausführungsform ist eine Mischung aus ätherischen Ölen als ätherisches Lösungsmittel bevorzugt, da ein Bouquet aus verschiedenen Düften als besonders angenehm wahrgenommen wird und auf diese Weise die Anwendung durch den Anwender unterstützt. Bevorzugt sind Mischungen innerhalb des ätherischen Lösungsmittels in denen Lavendel den größten Anteil in dem ätherischen Lösungsmittel ausmacht.

In einer anderen Ausführungsform ist nur Lavendel als ätherisches Öl in dem erfindungsgemäßen ätherischen Lösungsmittel vorhanden, da Lavendel durch seine beruhigende und sedierende Wirkung zu der erfindungsgemäßen Zusammensetzung unterstützend wirkt. Darüber hinaus gewährleistet die Verwendung von nur einem ätherischen Öl eine bessere Modulation der Wirkung und/oder eine höhere Verträglichkeit durch den Anwender.

In einer weiteren Ausführungsform umfasst das ätherische Öl oder die Mischung aus ätherischen Ölen für das ätherische Lösungsmittel *Lavandula angustifolia. Lavandula angustifolia* ist als spezielle Lavendelspezies bekannt für eine beruhigende und/oder sedierende Wirkung. Die Wirkung wird speziell durch die Mischung aus den Inhaltsstoffen, umfassend Monoterpenol-Ester, die in einer Ausführungsform umfasst sein können, erzielt. Bevorzugt ist für den möglichen Lavendelanteil nur *Lavandula angustifolia* oder eine etherischen Öl-Mischung aus Lavendel umfassend *Lavandula angustifolia* in der erfindungsgemäßen Gesamtmischung vorhanden. In einer ätherischen Öl-Mischung mit möglichem Lavendelanteil ist der Anteil an *Lavandula angustifolia* bevorzugt größer als der Anteil anderer Lavendelspezies.

Erfindungsgemäß ist das ätherische Lösungsmittel zu maximal 50 Gew.-% und mindestens 1 Gew.-%, bevorzugt zu maximal 45 Gew.-% und mindestens 1 Gew.-%, bevorzugt zu maximal 40 Gew.-% und mindestens 1 Gew.-% bevorzugt zu maximal 35 Gew.-% und mindestens 1 Gew.-%, bevorzugt zu maximal 30 Gew.-% und mindestens 1 Gew.-%, bevorzugt zu maximal 20 Gew.-% und mindestens 1 Gew.-%, bevorzugt zu maximal 10 Gew.-% und mindestens 1 Gew.-%, bevorzugt zu maximal 5 Gew.-% und mindestens 1 Gew.-%, bevorzugt zu maximal 1 Gew.-% und mindestens 1 Gew.-% , jeweils bezogen auf das Gesamtgewicht der Zusammensetzung, in der erfindungsgemäßen Zusammensetzung vorhanden.

In einer weiteren Ausführungsform ist das ätherische Lösungsmittel zu maximal 50 Gew.-% und mindestens 5 Gew.-%, bevorzugt zu maximal 45 Gew.-% und mindestens 5 Gew.-%, bevorzugt zu maximal 40 Gew.-% und mindestens 5 Gew.-% bevorzugt zu maximal 35 Gew.-% und mindestens 5 Gew.-%, bevorzugt zu maximal 30 Gew.-% und mindestens 5 Gew.-%, bevorzugt zu maximal 20 Gew.-% und mindestens 5 Gew.-%, bevorzugt zu maximal 10 Gew.-% und mindestens 5 Gew.-%, bevorzugt zu maximal 5 Gew.-% und mindestens 5 Gew.-%, jeweils bezogen auf das Gesamtgewicht der Zusammensetzung, in der erfindungsgemäßen Zusammensetzung vorhanden.

In einer Ausführungsform ist das ätherische Lösungsmittel zu maximal 50 Gew.-% und mindestens 10 Gew.-%, bevorzugt zu maximal 45 Gew.-% und mindestens 10 Gew.-%, bevorzugt zu maximal 40 Gew.-% und mindestens 10 Gew.-% bevorzugt zu maximal 35 Gew.-% und mindestens 10 Gew.-%, bevorzugt zu maximal 30 Gew.-% und mindestens 10 Gew.-%, bevorzugt zu maximal 20 Gew.-% und mindestens 10 Gew.-%, bevorzugt zu maximal 10 Gew.-% und mindestens 10 Gew.-%, jeweils bezogen auf das Gesamtgewicht der Zusammensetzung, in der erfindungsgemäßen Zusammensetzung vorhanden.

In einer weiteren Ausführungsform ist das ätherische Lösungsmittel zu maximal 50 Gew.-% und mindestens 20 Gew.-%, bevorzugt zu maximal 45 Gew.-% und mindestens 20 Gew.-%, bevorzugt zu maximal 40 Gew.-% und mindestens 20 Gew.-% bevorzugt zu maximal 35 Gew.-% und mindestens 20 Gew.-%, bevorzugt zu maximal 30 Gew.-% und mindestens 20 Gew.-%, bevorzugt zu maximal 20 Gew.-% und mindestens 20 Gew.-%, jeweils bezogen auf das Gesamtgewicht der Zusammensetzung, in der erfindungsgemäßen Zusammensetzung vorhanden.

In einer weiteren Ausführungsform ist das ätherische Lösungsmittel zu maximal 50 Gew.-% und mindestens 30 Gew.-%, bevorzugt zu maximal 45 Gew.-% und mindestens 30 Gew.-%, bevorzugt zu maximal 40 Gew.-% und mindestens 30 Gew.-% bevorzugt zu maximal 35 Gew.-% und mindestens 30 Gew.-%, bevorzugt zu maximal 30 Gew.-% und mindestens 30 Gew.-% in der erfindungsgemäßen Zusammensetzung, jeweils bezogen auf das Gesamtgewicht der Zusammensetzung, vorhanden.

In einer weiteren Ausführungsform ist das ätherische Lösungsmittel zu maximal 50 Gew.-% und mindestens 35 Gew.-%, bevorzugt zu maximal 45 Gew.-% und mindestens 35 Gew.-%, bevorzugt zu maximal 40 Gew.-% und mindestens 35 Gew.-%, bevorzugt zu maximal 35 Gew.-% und mindestens 35 Gew.-%, jeweils bezogen auf das Gesamtgewicht der Zusammensetzung, in der erfindungsgemäßen Zusammensetzung vorhanden.

In einer weiteren Ausführungsform ist das ätherische Lösungsmittel zu maximal 50 Gew.-% und mindestens 40 Gew.-%, bevorzugt zu maximal 45 Gew.-% und mindestens 40 Gew.-%, bevorzugt zu maximal 40 Gew.-% und mindestens 40 Gew.-%, jeweils bezogen auf das Gesamtgewicht der Zusammensetzung, in der erfindungsgemäßen Zusammensetzung vorhanden.

In jeder der vorangegangenen Aspekte ist die Ausführungsform bevorzugt, in der das ätherische Lösungsmittel maximal zu 10 Gew. -% und mindestens zu 40 Gew. -%, jeweils bezogen auf das Gesamtgewicht der Zusammensetzung, in der erfindungsgemäßen Zusammensetzung vorhanden ist.

In jeder der vorangegangenen Aspekte ist die Ausführungsform bevorzugt, in der das ätherische Lösungsmittel zu 10 Gew. -% oder zu 40 Gew. -%, jeweils bezogen auf das Gesamtgewicht der Zusammensetzung, in der erfindungsgemäßen Zusammensetzung vorhanden ist.

In einer weiteren Ausführungsform ist das Verhältnis der Verbindung 2,4,6-Trimethyl-4-phenyl-1,3-dioxan zu dem ätherischen Lösungsmittel 9 zu 1, bevorzugt 8 zu 2, bevorzugter 7 zu 3, noch bevorzugter 6 zu 4 gemessen an dem Anteil der beiden Komponenten.

In einer weiteren Ausführungsform umfassend alle vorangegangenen Aspekte besteht die erfindungsgemäße Zusammensetzung aus 2,4,6-Trimethyl-4-phenyl-1,3-dioxan und 1 bis 50 Gew.-% Lavendelöl als ätherisches Lösungsmittel. Alle Aspekte der Ausführungsformen bezüglich der erfindungsgemäßen Zusammensetzung entsprechen ebenfalls Ausführungsformen des Verfahrens.

In einer Ausführungsform kann das ätherische Lösungsmittel aus sekundären Pflanzenstoffen durch Wasserdampfdestillation, Kaltpressung und/oder Extraktion erfolgen, wobei die Extraktion bevorzugt ist. Speziell durch Extraktion können ätherische Öle vor allem aus Blüten gewonnen werden, ermöglichen den Erhalt besonders vieler Inhaltsstoffe gegenüber anderen Verfahren. Die Pflanzeninhaltsstoffe für das ätherische Lösungsmittel können aus Stängel, Blatt, Wurzel, etc., gewonnen werden. Hierbei ist klar zu verstehen, dass die möglichen Pflanzeninhaltsstoffe aus Pflanzen nicht auf diese Aufzählungen begrenzt sind.

In einer Ausführungsform werden die erfindungsgemäßen Pflanzeninhaltsstoffe direkt aus der Pflanze oder aus den Pflanzenteilen gewonnen, oder die Pflanzeninhaltsstoffe werden aus der Pflanze oder den Pflanzenteilen durch den Fachmann bekannte Verfahren in Ölen gebunden, oder die Pflanzeninhaltsstoffe werden synthetisch hergestellt.

In einer anderen Ausführungsform wird die Konzentration der wichtigen Bestandteile des ätherischen Öls bestimmt. Bestimmungsmöglichkeiten sind dem Fachmann bekannt. Wichtige Bestandteile sind verschiedene Terpene, Aromate, etc.

In einem weiteren Aspekt der Ausführungsform bezüglich des Verfahrens werden die Bestandteile entweder angepasst, oder die ätherischen Öle aussortiert, deren Anteil an wichtigen Bestandteilen zu hoch oder zu niedrig für die erfindungsgemäße Zusammensetzung ist. Somit kann eine entsprechend konsistente Qualität und Sicherheit des Schlafdufts gewährleistet werden.

Alle Aspekte der Ausführungsformen bezüglich der erfindungsgemäßen Zusammensetzung und des Verfahrens sind ebenfalls Ausführungsformen bzgl. der Verwendung. Umfasst ist auch eine Ausführungsform zur Verwendung der erfindungsgemäßen Zusammensetzung zur Prophylaxe und/oder Therapie von Depressionen, Unruhe- und Angstzuständen, Einschlafproblemen und/oder Durchschlafproblemen, und/oder Migräne, bevorzugt zur Prophylaxe und/oder Therapie von Einschlafproblemen und/oder Durchschlafprobleme.

Umfasst ist auch eine Ausführungsform zur Verwendung der erfindungsgemäßen Zusammensetzung in einer therapeutisch wirksamen Menge zur Prophylaxe und/oder Therapie von Depressionen, Unruhe- und Angstzuständen, Einschlafproblemen und/oder Durchschlafproblemen, und/oder Migräne, bevorzugt zur Prophylaxe und/oder Therapie von Einschlafproblemen und/oder Durchschlafprobleme.

Wie hierin verwendet, bezieht sich der Begriff "therapeutisch wirksame Menge" auf eine Menge, die zur Vorbeugung oder Behandlung von Schlafstörungen durch die verwendeten einzelnen Komponenten und/oder durch die verwendete erfindungsgemäße Zusammensetzung wirksam ist.

Umfasst ist auch eine Ausführungsform zur Verwendung durch Applikation der erfindungsgemäßen Zusammensetzung mittels eines Sprays durch den Anwender bevorzugt auf ein Kissen, ein Tuch/Schlaftuch, ein Pyjama umfasst.

Umfasst ist auch eine Ausführungsform zur Verwendung durch Applikation der erfindungsgemäßen Zusammensetzung mittels eines Nasenclips oder Nasenpflasters durch den Anwender kurz vor dem Schlafprozess umfasst.

Bevorzugt umfasst ist eine Ausführungsform zur Verwendung durch Applikation der erfindungsgemäßen Zusammensetzung durch Auslegen des Tuchs mit der erfindungsgemäßen Zusammensetzung durch den Anwender kurz vor dem Schlafprozess umfasst, da so eine regulierte Menge in einer durch den Vliesstoff gewährleisteten optimalen, zeitlich regulierten Abgabe der erfindungsgemäßen Zusammensetzung gewährleistet werden kann

Die vorliegende Erfindung stellt auch ein Verfahren zur Vorbeugung oder Behandlung beispielsweise von Einschlafproblemen und Durchschlafproblemen dar, wobei das Verfahren die Verabreichung der erfindungsgemäßen Zusammensetzung umfasst.

Bevorzugt sind Säugetiere, insbesondere Menschen.

Unter dem nicht-medizinischen Gesichtspunkt ist im Rahmen der vorliegenden Erfindung insbesondere eine Mischung aus 2,4,6-Trimethyl-4-phenyl-1,3-dioxan und Lavendelöl als erfindungsgemäße Zusammensetzung bevorzugt. Diese Mischung wird von den Anwendern als angenehm im Geruch aufgefasst und unterstützt aufgrund des angenehmen Geruchs insbesondere den Einschlafprozess.

Die erfindungsgemäße Mischung aus 2,4,6-Trimethyl-4-phenyl-1,3-dioxan und Lavendelöl wird vorzugsweise in Abmischungen mit den nachfolgenden Mengen verwendet:

| | 2,4,6-Trimethyl-4-phenyl-1,3-dioxan | Lavendelöl |
|---|---|---|
| Erste Ausführungsform | 0,1 bis 5 Gew.-% | 1 bis 50 Gew.-% |
| Zweite Ausführungsform | 0,5 bis 4,5 Gew.-% | 3 bis 40 Gew.-% |
| Dritte Ausführungsform | 1 bis 4 Gew.-% | 8 bis 35 Gew.-% |
| Vierte Ausführungsform | 1,5 bis 3,5 Gew.-% | 12 bis 30 Gew.-% |
| Fünfte Ausführungsform | 2 bis 3 Gew.-% | 15 bis 25 Gew.-% |

In den vorstehend beschriebenen fünf Ausführungsformen werden zusätzlich nehmen den beiden Bestandteilen 2,4,6-Trimethyl-4-phenyl-1,3-dioxan und Lavendelöl noch ein geruchsneutrales Füllöl, insbesondere Triethylcitrat, verwendet.

Zusätzlich können in den vorstehend genannten Ausführungsformen noch *Passiflora incarnata* verwendet werden.

In den fünf vorstehend beschriebenen Ausführungsformen wird *Passiflora incarnata* in Mengen zwischen 1 und 50 Gew.-%, bevorzugt zwischen 2 und 40 Gew.-%, noch weiter bevorzugt 3 bis 30 Gew.-%, verwendet.

Die fünf vorstehend genannten Ausführungsformen sind in der Reihenfolge erste Ausführungsform, zweite Ausführungsform, dritte Ausführungsform, vierte Ausführungsform und fünfte Ausführungsform bevorzugt, wobei die fünfte Ausführungsform die bevorzugte Ausführungsform unter Berücksichtigung der Riechqualität von Duftstoffen zum Schlafen ist.

Nachfolgend wird die vorliegende Erfindung anhand von Beispielen und Versuchsbeispielen im Detail beschrieben. Es ist jedoch zu verstehen, dass diese Beispiele nur zum besseren Verständnis der vorliegenden Erfindung dienen und nicht dazu gedacht sind, den Umfang der vorliegenden Erfindung zu begrenzen.

### BEISPIELE

Nicht-medizinische Verwendung der erfindungsgemäßen Geruchszusammensetzung zur Verbesserung der Riechqualität von Duftstoffen:
Es werden insgesamt sechs Mischungen gemäß den nachfolgenden Tabellen aus 2,4,6-Trimethyl-4-phenyl-1,3-dioxan und Lavendelöl in Abmischung mit *Passiflora incarnata* und Füllöl (Triethylcitrat) durch einfaches physikalisches Mischen der Bestandteile hergestellt:

| Bsp. | 1* | 2* | 3* | 4* | 5 | 6 |
|---|---|---|---|---|---|---|
| VC | 100 | - | 90 | 40 | 2,5 | 2,5 |
| LÖ | - | 100 | 5 | 30 | 40 | 20 |
| PI | - | - | 5 | 30 | 5 | 15 |
| FÖ | - | - | - | - | 52,5 | 62,5 |

| | | | | | | |
|---|---|---|---|---|---|---|
| * nicht erfindungsgemäß | | | | | | |

### Bedeutungen:

- VC:: Vertacetal Coeur (2,4,6-Trimethyl-4-phenyl-1,3-dioxan), erhältlich von der Firma Symrise AG, Holzminden, Deutschland
- LÖ:: Lavendelöl (Lavendula angustofilia, erhältlich von der Firma Payan Bertrand S.A., Frankreich, als Lavender Oil extra
- PI:: Passiflora incarnata (Passionsblumenöl)
- FÖ:: Füllöl (Triethylcitrat), erhältlich von der Firma Payan Bertrand S.A., Frankreich, als Citroflex

Die mit diesen sechs Mischungen erhaltenen Duftqualitäten wurden von fünf Probanden unabhängig voneinander mit Schulnoten bewertet, wobei eine höhere Schulnote eine schlechtere Bewertung als eine niedrige Schulnote darstellt:

| Testperson | Bsp. 1* | Bsp. 2* | Bsp. 3* | Bsp. 4* | Bsp. 5 | Bsp. 6 |
|---|---|---|---|---|---|---|
| 1 | 4,0 | 3,5 | 4,0 | 3,0 | 1,5 | 1,0 |
| 2 | 5,0 | 4,0 | 3,5 | 3,5 | 2,0 | 1,0 |
| 3 | 5,0 | 4,0 | 3,5 | 3,5 | 1,5 | 1,5 |
| 4 | 4,5 | 3,5 | 2,5 | 2,0 | 2,0 | 1,0 |
| 5 | 4,5 | 3,5 | 3,0 | 2,0 | 1,5 | 1,0 |
| Gesamt | 4,6 | 3,7 | 3,3 | 2,8 | 1,7 | 1,1 |
| Bewertung | schlecht | nicht gut | wenig besser | besser | Gut | am besten |

| | | | | | | |
|---|---|---|---|---|---|---|
| * nicht erfindungsgemäß | | | | | | |

Aus den vorstehenden Ergebnissen zeigt sich, dass die Zusammensetzung aus
20 Gew.-% Lavendelöl, und
2,5 Gew.-% 2,4,6-Trimethyl-4-phenyl-1,3-dioxan
am angenehmsten von den Probanden aufgefasst und beurteilt wird.

### Medizinische Verwendung:

Jeder Verweis auf therapeutische oder chirurgische Behandlungsverfahren in dieser Beschreibung ist als Verweis auf Verbindungen, pharmazeutische Zubereitungen und Arzneimittel zur Anwendung in diesen Verfahren zu verstehen.

Die Auswirkungen verschiedener Verhältnisse innerhalb der erfindungsgemäßen Zusammensetzungen (d.h. unterschiedlicher Konzentrationen von 2,4,6-Trimethyl-4-phenyl-1,3-dioxan und einem ätherischen Lösungsmittel) auf den Schlaf wurden durch Messung der polysomnographischen (PSG) und subjektiven Schlafqualität untersucht. Dreißig Erwachsene (fünfzehn Frauen: im Alter von 35-63 Jahren: Durchschnittsalter 47 ± 8 Jahre) wurden in einem Schlaflabor über einen Zeitraum von vier Nächten (Bedingungen) beobachtet: In einer der Nächte haben die Probanden unter Bedingung 1 (ohne Verwendung eines Schlafdufts) geschlafen. In einer anderen Nacht haben die Probanden unter Bedingung 2 (Verwendung einer Schlafduftmischung aus 90% 2,4,6-Trimethyl-4-phenyl-1,3-dioxan, 10% ätherisches Lösungsmittel) geschlafen. In einer weiteren Nacht haben die Probanden unter Bedingung 3 (Verwendung einer Schlafduftmischung aus 60% 2,4,6-Trimethyl-4-phenyl-1,3-dioxan, 40% ätherisches Lösungsmittel) geschlafen. Die verschiedenen Gerüche wurden während der Nacht an der Oberbekleidung des Oberkörpers aufgetragen. Alle Versuchspersonen haben unter typischer Schlaflosigkeit bzw. Schlaferhaltungsinsomnie gelitten. Typische Schlaflosigkeit umfasst Schlafprobleme bzgl. des Einschlafens bzw. Durchschlafens, die weniger als dreimal pro Woche auftreten.

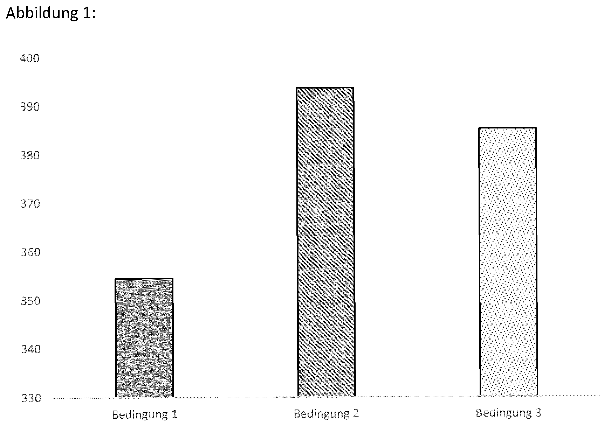

Zu den in dieser Studie gemessenen PSG-Parametern gehören die Latenzzeit bis zum Einsetzen der ersten Schlafepoche (SO), die Latenzzeit bis zum anhaltenden Schlaf (LPS), die Wachheit nach Einsetzen des Schlafs (WASO) und die Gesamtschlafzeit (TST). Die Abbildungen 1, 2 und 3 zeigen die Dauer (in Min.) jedes PSG-Parameters (y-Achse) in Abhängigkeit von allen drei Bedingungen (x-Achse). Zu beachten sind die verschiedenen Werte der y-Achse aufgrund der unterschiedlichen Bereiche der gemessenen Parameter.

Abbildung 1 zeigt die Gesamtschlafzeit (TST). Bei TST werden hohe Werte durch eine längere Schlafdauer, bzw. eine kürzere Einschlafdauer bedingt. Entsprechend zeigt sich eine längere Schlafdauer bei den Bedingung 2 und 3. Die Gesamtzeit der Bedingung 3 ist lediglich um 2,3% verkürzt gegenüber der Bedingung 2, obwohl die Bedingung 3 30% weniger 2,4,6-Trimethyl-4-phenyl-1,3-dioxan enthält.

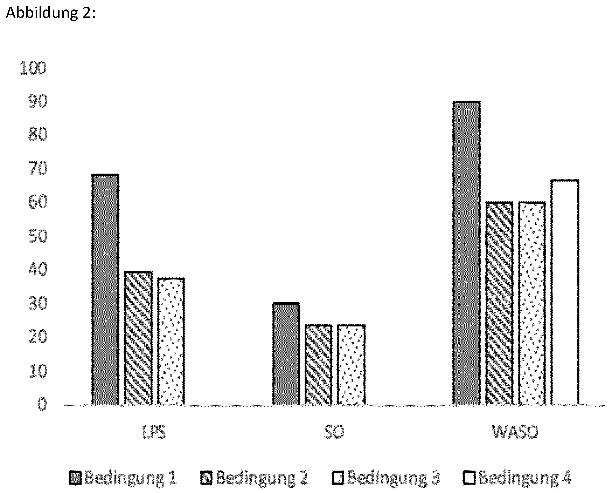

In Abbildung 2 werden LPS, SO und WASO miteinander verglichen. Bei den Parametern LPS, SO und WASO werden hohe Werte durch kürzere Schlafdauer, bzw. eine längere Einschlafdauer bedingt. Die Messungen für die Bedingungsfaktoren zeigen durchweg bessere Ergebnisse für die Anwendung von Bedingung 2 bzw. Bedingung 3 im Vergleich zu Bedingung 1. Bei dem Parameter SO und WASO entspricht die Gesamtzeit der Bedingung 3 der Gesamtzeit der Bedingung 2, obwohl die Bedingung 3 30% weniger 2,4,6-Trimethyl-4-phenyl-1,3-dioxan enthält. Entsprechend wird überraschend, trotz quantitativer Reduzierung an 2,4,6-Trimethyl-4-phenyl-1,3-dioxan, qualitativ denselben Effekt durch Bedingung 3 erzielt. Bei dem Parameter LPS weicht die Gesamtzeit der Bedingung 3 lediglich um ca. 5% von der Bedingung 2 ab, obwohl die Bedingung 3 30% weniger 2,4,6-Trimethyl-4-phenyl-1,3-dioxan enthält. Auch hier führt die quantitative Reduzierung an 2,4,6-Trimethyl-4-phenyl-1,3-dioxan zu einem qualitativ unerwartet hohen Ergebnis.

Alle PSG-Parameter zeigen eine signifikant schlechtere Schlafqualität in der Bedingung 1 im Vergleich zu den Bedingungen 2 und 3. Der Vergleich zwischen den PSG-Parametern der Bedingungen 2 und 3 ergeben keinen signifikanten Unterschied bzgl. des Effekts innerhalb dieser beiden Gruppen.

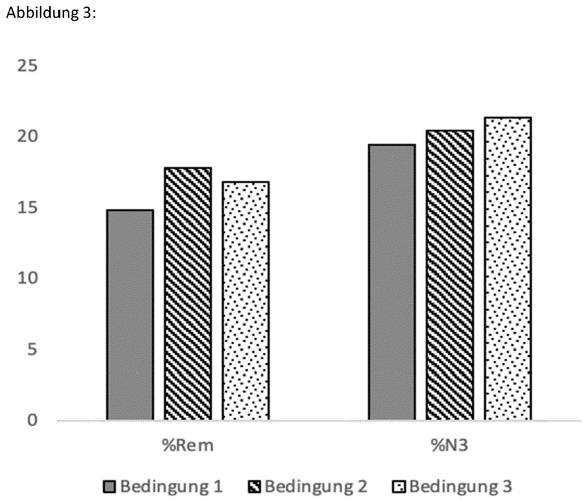

Das Gleiche gilt für den Vergleich des prozentualen REM-Schlafs (Rapid Eye Movement) von TST (%REM) aus Abbildung 3. %REM ist in der ersten Bedingung im Vergleich zu den Bedingungen 2 und 3 am niedrigsten. Der Anteil der Tiefschlafphase N3 (traumlose Tiefschlafphase) der TST (%N3) ist in Bedingung 1 verkürzt gegenüber den Bedingungen 2 und 3.

Die PSG-Ergebnisse deuten entsprechend auf eine gleiche Schlafqualität der verschieden anteiligen Zusammensetzungen auf objektive Schlafparameter wie TST, WASO, SO und LPS hin. Der Einfluss auf die Schlafqualität, gemessen durch PSG, unterscheidet sich nicht signifikant zwischen Bedingung 2 und Bedingung 3. Überraschend hat sich somit herausgestellt, dass die Verwendung von 60% 1,3-Dioxan, bevorzugt 2,4,6-Trimethyl-4-phenyl-1,3-dioxan ebenso effektiv ist, wie die Verwendung von 90% 2,4,6-Trimethyl-4-phenyl-1,3-dioxan.

Zusätzlich wurde der Eindruck der Probanden bezüglich der Verwendungsform bewertet. Hierfür wurde ein Placebo nur aus Duft bestehend (Bedingung 4) gegenüber Bedingung 2 und 3 bewertet. Die Probanden haben aus einer Liste ausgewählt, umfassend "über Nacht auf einer Mund-Nasen-Maske tragen", "auf ein Schlaftuch träufeln", "auf den Pyjama träufeln", "aufs Kopfkissen träufeln", "auf einen Nasen-Clip tragen", "direkt auf der Haut tragen", "wiederverwenden", wie Sie die Zusammensetzung am ehesten Verwenden. Mehrfachnennungen waren möglich.

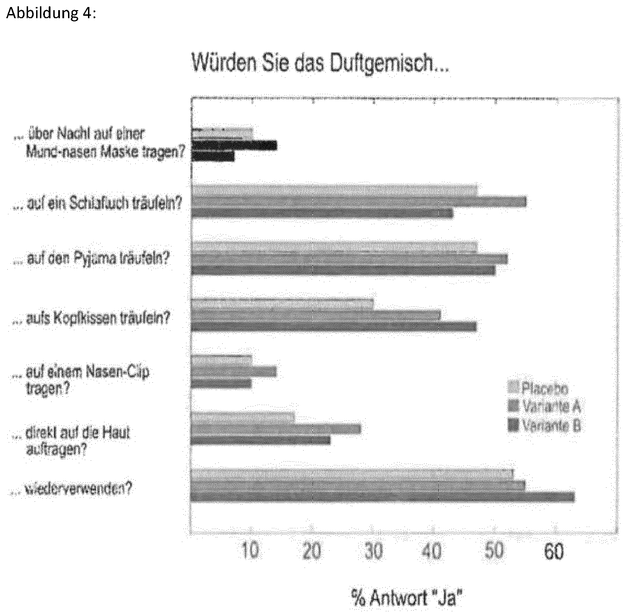

Abbildung 4 zeigt deutlich einen Vorteil von Bedingung 3 gegenüber Bedingung 4 und Bedingung 2. Bedingung 3 hat insgesamt die meisten positiven Antworten als Verwendungsform insgesamt bekommen. Weiterhin hat über die Hälfte der Probanden, dass würden sie die bereitgestellte erfindungsgemäße Zusammensetzung wiederverwenden würden. Über 50% der Probanden bevorzugen die Aufnahme der Zusammensetzung über den Schlafanzug, oder über ein Schlaftuch. Die Anwendung aller Bedingungen in der Nähe der Atemwege und direkt auf der Haut wird von der deutlichen Mehrheit eher abgelehnt.

Insgesamt zeigen die PSG-Ergebnisse einen Vorteil bzgl. der Bedingungen 2 und 3 gegenüber der Bedingung 1. Die Bewertung zur Anwendung durch die Probanden zeigt einen leichten Vorteil zugunsten von Bedingung 3.

## Patentansprüche

1. Zusammensetzung, umfassend 0,1 bis 5 Gew.-% 2,4,6-Trimethyl-4-phenyl-1,3-dioxan und 1 bis 50 Gew.-% Lavendelöl als ätherisches Lösungsmittel.

2. Zusammensetzung nach Anspruch 1, **dadurch gekennzeichnet, dass** die Zusammensetzung zusätzlich Passionsblumenöl als ätherisches Lösungsmittel umfasst.

3. Zusammensetzung nach Anspruch 1 oder 2, **dadurch gekennzeichnet, dass** die Zusammensetzung auf einem Vliestuch aufgetragen ist.

4. Verfahren zur Herstellung einer Zusammensetzung gemäß einem oder mehreren der Ansprüche 1 bis 3, **dadurch gekennzeichnet, dass** man die Verbindung 2,4,6-Trimethyl-4-phenyl-1,3-dioxan mit dem ätherischen Lösungsmittel mischt.

5. Zusammensetzung gemäß einem der Ansprüche 1 bis 3 zur Verwendung bei der Prophylaxe und/oder Therapie von Depressionen, Unruhe- und Angstzuständen, Einschlafproblemen und/oder Durchschlafproblemen, und/oder Migräne, bevorzugt zur Prophylaxe und/oder Therapie von Einschlafproblemen und/oder Durchschlafproblemen.

6. Zusammensetzung nach Anspruch 5, zur Verwendung bei der Prophylaxe und/oder Therapie von Depressionen, Unruhe- und Angstzuständen, Einschlafproblemen und/oder Durchschlafproblemen, und/oder Migräne, bevorzugt zur Prophylaxe und/oder Therapie von Einschlafproblemen und/oder Durchschlafproblemen,
durch Applikation der erfindungsgemäßen Zusammensetzung mittels eines Sprays auf ein Kissen, ein Tuch/Schlaftuch und/oder einem Pyjama, oder
durch Applikation der Zusammensetzung mittels eines Nasenclips oder Nasenpflasters vor dem Schlafprozess, oder
durch Auslegen des Tuchs mit der Zusammensetzung kurz vor dem Schlafprozess.

7. Verwendung einer Zusammensetzung gemäß einem der Ansprüche 1 bis 3 zur Verbesserung des Schlafens.

## Claims

1. A composition comprising 0.1 to 5% by weight of 2,4,6-trimethyl-4-phenyl-1,3-dioxane and 1 to 50% by weight of lavender oil as an ethereal solvent.

2. The composition according to claim 1, **characterized in that** the composition additionally comprises passion flower oil as an ethereal solvent.

3. The composition according to claim 1 or 2, **characterized in that** the composition is applied to a non-woven cloth.

4. A process for preparing a composition according to one or more of claims 1 to 3, **characterized in that** the compound 2,4,6-trimethyl-4-phenyl-1,3-dioxane is mixed with the an ethereal solvent.

5. Composition according to one of claims 1 to 3 for use in the prophylaxis and/or therapy of depression, restlessness and anxiety states, problems falling asleep and/or problems sleeping through the night, and/or migraine, preferably for the prophylaxis and/or therapy of problems falling asleep and/or problems sleeping through the night.

6. Composition according to claim 5, for use in the prophylaxis and/or therapy of depression, restlessness and anxiety states, problems falling asleep and/or problems sleeping through the night, and/or migraine, preferably for the prophylaxis and/or therapy of problems falling asleep and/or problems sleeping through the night,
by application of the composition according to the invention by means of a spray onto a pillow, a sheet/sleeping towel and/or pyjamas, or
by applying the composition by means of a nose clip or nose patch before the sleep process, or
by laying out the cloth with the composition shortly before the sleep process.

7. Use of a composition according to any one of claims 1 to 3 for improving sleep.

## Revendications

1. Composition comprenant de 0,1 à 5 % en poids de 2,4,6-triméthyl-4-phényl-1,3-dioxane et de 1 à 50 % en poids d'huile de lavande comme solvant éthéré.

2. Composition selon la revendication 1, **caractérisée en ce que** la composition comprend en outre de l'huile de passiflore comme solvant éthéré.

3. Composition selon la revendication 1 ou 2, **caractérisée en ce que** la composition est appliquée sur une toile non tissée.

4. Procédé de préparation d'une composition selon l'une ou plusieurs des revendications 1 à 3, **caractérisé en ce que** l'on mélange le composé 2,4,6-triméthyl-4-phényl-1,3-dioxane avec le solvant éthéré.

5. Composition selon l'une des revendications 1 à 3 pour l'utilisation dans la prophylaxie et/ou la thérapie de la dépression, des états d'agitation et d'anxiété, des problèmes d'endormissement et/ou des problèmes de maintien du sommeil, et/ou de la migraine, de préférence dans la prophylaxie et/ou la thérapie des problèmes d'endormissement et/ou des problèmes de maintien du sommeil.

6. Composition selon la revendication 5, pour l'utilisation dans la prophylaxie et/ou la thérapie de la dépression, des états d'agitation et d'anxiété, des problèmes d'endormissement et/ou des problèmes de maintien du sommeil, et/ou de la migraine, de préférence pour la prophylaxie et/ou la thérapie des problèmes d'endormissement et/ou des problèmes de maintien du sommeil,
par application de la composition selon l'invention au moyen d'un spray sur un oreiller, une toile/toile de sommeil et/ou un pyjama, ou
par application de la composition au moyen d'un pince-nez ou d'un patch nasal avant le processus de sommeil, ou
par étalage de la toile avec la composition juste avant le processus de sommeil.

7. Utilisation d'une composition selon l'une des revendications 1 à 3 pour améliorer le sommeil.
